(19) Europäisches Patentamt / European Patent Office / Office européen des brevets

(11) **EP 3 377 559 B1**

(12) **EUROPÄISCHE PATENTSCHRIFT**

(45) Veröffentlichungstag und Bekanntmachung des Hinweises auf die Patenterteilung:
**17.06.2020 Patentblatt 2020/25**

(21) Anmeldenummer: **16779056.7**

(22) Anmeldetag: **06.10.2016**

(51) Int Cl.:
**C08G 77/04** (2006.01)     **C08G 77/06** (2006.01)
**C08G 77/08** (2006.01)     **A61K 8/89** (2006.01)
**A61K 8/891** (2006.01)     **C08G 77/18** (2006.01)

(86) Internationale Anmeldenummer:
**PCT/EP2016/073902**

(87) Internationale Veröffentlichungsnummer:
**WO 2018/065058 (12.04.2018 Gazette 2018/15)**

(54) **VERFAHREN ZUR HERSTELLUNG SPHÄRISCHER POLYSILSESQUIOXANPARTIKEL**

METHOD FOR PRODUCING SPHERICAL POLYSILSESQUIOXANE PARTICLES

PROCÉDÉ POUR PRODUIRE DES PARTICULES SPHÉRIQUES DE POLYSILSESQUIOXANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.09.2018 Patentblatt 2018/39**

(73) Patentinhaber: **Wacker Chemie AG**
**81737 München (DE)**

(72) Erfinder: **KNÖR, Sebastian**
**84547 Emmerting (DE)**

(74) Vertreter: **Fritz, Helmut et al**
**Wacker Chemie AG**
**Zentralbereich Patente, Marken und Lizenzen**
**Hanns-Seidel-Platz 4**
**81737 München (DE)**

(56) Entgegenhaltungen:
- **DATABASE WPI Week 199817 Thomson Scientific, London, GB; AN 1998-189384 XP002763903, -& JP H10 45914 A (TOSHIBA SILICONE KK) 17. Februar 1998 (1998-02-17)**
- **DATABASE WPI Week 200422 Thomson Scientific, London, GB; AN 2004-230738 XP002763904, -& JP 2003 335860 A (TOSHIBA SILICONE KK) 28. November 2003 (2003-11-28)**
- **DATABASE WPI Week 200055 Thomson Scientific, London, GB; AN 2000-581615 XP002763905, -& JP 2000 186148 A (TOSHIBA SILICONE KK) 4. Juli 2000 (2000-07-04) & JP 3 970449 B2 5. September 2007 (2007-09-05) in der Anmeldung erwähnt**

EP 3 377 559 B1

**Beschreibung**

Die Erfindung betrifft ein Verfahren zur Herstellung sphärischer Polysilsesquioxanpartikel durch Hydrolyse von Trialkoxysilan, Kondensation des Hydrolysats und Sprühtrocknung der Partikel und die Polysilsesquioxanpartikel.

Der Stand der Technik, beispielsweise JP3970449B2, JPH1045914A, JP2003335860A, JPH06248081A und JPH0488023A kennt verschiedene Verfahren zur Herstellung von sphärischen Polymethylsilsesquioxanpartikeln. JP3970449B2 beschreibt die Optimierung der Raum-Zeit-Ausbeute und der Kontrolle der Partikelgröße. Bei der Trocknung kommt es zu einer Verschmelzung der Partikel und den Aufbau einer Netzwerkstruktur. Zur Erlangung pulverförmiger Produkte werden die verschmolzenen Partikel umständlich durch anschließende Mahlung erhalten. Nachteilig ist der erforderliche Arbeitsschritt der Mahlung, welcher teuer und arbeitsaufwändig ist. Die sehr voluminösen und staubigen Polysilsesquioxanpartikel müssen nach dem Trocknen zunächst abgefüllt werden. Dies bedeutet Materialverlust und Reinigungsaufwand der Abfüllanlage. Danach muss das Material zur Mühle transportiert werden. Aufgrund der geringen Schüttdichte der Polysilsesquioxanpartikel werden sehr große Gebinde und benötigt, was den Transport und die Zwischenlagerung aufwändig und teuer macht. An der Mühle muss das Material erneut umgefüllt werden. Dies bedeutet erneut Materialverluste, außerdem müssen Schutzmaßnahmen zum Schutz der Mitarbeiter vor dem zwar nicht toxischen, aber sehr staubigen Material getroffen werden. Es kommt erneut zu Materialverlusten in der Mühle durch Abrieb oder gebrochene Partikel, aufgrund der vergleichsweise harschen Bedingungen. Bisher ist kein Verfahren beschrieben, welches eine Kontrolle des Agglomerisationsverhaltens der Partikel erlaubt.

Gegenstand der Erfindung ist ein Verfahren zur Herstellung sphärischer Polysilsesquioxanpartikel, bei dem in einem ersten Schritt Trialkoxysilan der allgemeinen Formel (I)

$$RSi(OR^1)_3 \qquad (I),$$

in der

R einen Kohlenwasserstoffrest mit 1 bis 16 Kohlenstoffatomen dessen Kohlenstoffkette durch nicht benachbarte Gruppen -O-unterbrochen sein kann,
$R^1$ einen $C_1$- bis $C_4$-Alkylrest bedeuten,
mit angesäuertem Wasser mit einem pH-Wert von höchstens 6 unter Durchmischung zu einem Hydrolysat umgesetzt wird,
in einem zweiten Schritt das Hydrolysat mit einer Lösung einer Base in Wasser oder $C_1$- bis $C_4$-Alkanol vermischt wird,
in einem dritten Schritt die Mischung mindestens 2 h aufbewahrt wird
und in einem vierten Schritt die Polysilsesquioxanpartikel sprühgetrocknet werden.

Es wurde gefunden, dass agglomerisationsfreie sphärische Polysilsesquioxanpartikel erhalten werden können, wenn die Partikel direkt aus der Dispersion mittels Sprühtrocknung isoliert werden. Dadurch werden desagglomerierte Partikel in nur einem Arbeitsgang erhalten.
Transport-, Arbeits- und Reinigungsintensives offenes Pulverhandling, welches für eine separate Trocknung und nachfolgende Mahlung notwendig ist, kann so vermieden werden. Die Polysilsesquioxanpartikel werden unter sehr milden Bedingungen dispergiert. Materialverlust durch Abrieb oder gebrochene Partikel, wie es bei der Mühle vorkommen kann, sind bei der Sprühtrocknung stark reduziert.

Solche Partikel zeigen ein sehr vorteilhaftes Verhalten, insbesondere für kosmetische Anwendungen. Sie gehen bereits bei geringer Scherung in einen Flüssigkeits-ähnlichen fließfähigen Zustand über (Fluidisieren) und sind dadurch außerordentlich leicht zu verteilen und bewirken ein samtiges Hautgefühl. Dieses Verhalten ist bei agglomerierten Partikeln nicht zu beobachten. Diese ballen beim Verteilen auf der Haut zusammen.

Fluides, also flüssigkeits-ähnliches Verhalten, zeigt sich insbesondere direkt nach dem Aufschütteln der Polysilsesquioxanpartikel. Das fluide Verhalten ist umso stärker ausgeprägt, umso stärker die Volumenzunahme ist. Ein Material, das 50% Volumenzunahme aufweist, zeigt bereits fluides Verhalten, was sich beispielsweise darin äußert, dass das Material im Gebinde - unmittelbar nach dem Aufschütteln - beim Schwenken des Gebindes ähnlich einer Flüssigkeit hin- und her fließt. Ein Material mit 50% Volumenzunahme sedimentiert sehr schnell und geht in den nichtfluiden Ausgangszustand zurück, was nachteilig ist. Die sphärischen Polysilsesquioxanpartikel zeigen vorzugsweise mindestens 100% Volumenzunahme.

Die Polysilsesquioxanpartikel weisen vorzugsweise mindestens 30 Gew.-%, bevorzugt mindestens 40 Gew.-%, besonders bevorzugt mindestens 50 Gew.-%, einer Siebfraktion <20 $\mu$m auf.

Die Polysilsesquioxanpartikel weisen vorzugsweise mindestens 60 Gew.-%, bevorzugt mindestens 70 Gew.-%, einer Siebfraktion <40 $\mu$m auf.

Die Polysilsesquioxanpartikel weisen vorzugsweise weniger als 25 Gew.-%, bevorzugt weniger als 20 Gew.-%, besonders bevorzugt weniger als 15 Gew.-%, einer Siebfraktion >100 $\mu$m auf.

R bedeutet vorzugsweise einen Alkylrest mit 1 bis 6 Kohlenstoffatomen oder Phenylrest, insbesondere Ethylrest oder Methylrest.

$R^1$ bedeutet vorzugsweise einen Methyl-, Ethyl-, oder n-Propylrest, insbesondere einen Methylrest.

Bevorzugte Trialkoxysilane der allgemeinen Formel (I) sind Methyltrimethoxysilan, Methyltriethoxysilan, Methyltri-n-propoxysilan, Methyltriisopropoxysilan und Methyltris(2-methoxyethoxy)silan und Gemische davon.

Die Umsetzung zu einem Hydrolysat erfolgt vorzugsweise in angesäuertem Wasser mit einem pH-Wert von höchstens 5,5 besonders bevorzugt höchstens 4,5 und vorzugsweise mindestens 1, besonders bevorzugt mindestens 2, insbesondere mindestens 2,3.

Das eingesetzte Wasser ist vorzugsweise entsalzt und weist vor dem Ansäuern vorzugsweise eine Leitfähigkeit von höchstens 50 µS/cm, bevorzugt höchstens 30 µS/cm, besonders bevorzugt höchstens 20 µS/cm auf, insbesondere bevorzugt höchstens 10 µS/cm auf, jeweils gemessen bei 20°C.

Zum Ansäuern des eingesetzten Wassers können Brønstedt-Säuren oder Lewis-Säuren eingesetzt werden. Beispiele für Lewis-Säuren sind $BF_3$, $AlCl_3$, $TiCl_3$, $SnCl_4$, $SO_3$, $PCl_5$, $POCl_3$, $FeCl_3$ und dessen Hydrate und $ZnCl_2$. Beispiele für Brønstedt-Säuren sind Salzsäure, Bromwasserstoffsäure, Schwefelsäure, Salpetersäure, salpetrige Säure, Chlorsulfonsäure, Phosphorsäuren, wie ortho-, meta- und Polyphosphorsäuren, Borsäure, selenige Säure, Salpetersäure, Carbonsäuren, wie Ameisensäure, Essigsäure, Propionsäure, Citronensäure und Oxalsäure, Halogenessigsäuren, wie Trichlor- und Trifluoressigsäure, p-Toluolsulfonsäure, saure Ionenaustauscher, saure Zeolithe und säureaktivierte Bleicherde.

Bevorzugt sind Salzsäure, Bromwasserstoffsäure und Essigsäure.

Das Ansäuern des Wassers kann vor der Umsetzung zum Hydrolysat gleichzeitig mit der Umsetzung erfolgen oder sowohl vor der Umsetzung als auch gleichzeitig mit der Umsetzung erfolgen. In einer besonderen Ausführungsform wird das Wasser teilweise vor der Umsetzung zum Hydrolysat mit Salzsäure angesäuert und ein weiterer Teil Salzsäure wird durch die Trialkoxysilane der allgemeinen Formel (I) eingebracht.

Die Hydrolyse des Trialkoxysilans ist eine schwach exotherme Reaktion. Die Temperatur im ersten Schritt wird in einer bevorzugten Ausführungsform gegebenenfalls durch Heizen oder Kühlen vorzugsweise bei 0°C bis 60°C, bevorzugt bei 10°C bis 50°C, besonders bevorzugt bei 15°C bis 40°C, ganz besonders bevorzugt bei 15 bis 30 °C, insbesondere bei 15 - 25 °C gehalten, wobei die Temperaturschwankung nach Erreichen der Zieltemperatur vorzugsweise weniger als 10 °C, bevorzugt weniger als 5 °C beträgt. Die Dosierung des Trialkoxysilans kann beliebig vor oder nach Erreichen der Zieltemperatur begonnen werden.

In einer anderen Ausführungsform wird das Trialkoxysilan in einer Portion zudosiert. Dabei wird die Wärme nicht aktiv oder nur teilweise herausgekühlt. In dieser Ausführungsform findet eine exotherme Zunahme der Temperatur nach Zugabe des Trialkoxysilans statt. Die Temperatur der Umsetzung im ersten Schritt beträgt 20 °C bis 80°C, vorzugsweise bis 60°C.

Vorzugsweise wird das Trialkoxysilan in 0,5 bis 5 h, besonders bevorzugt höchstens 2 h dosiert. Zwischen der schnellen Zugabe und der Dosierung gibt es einen fließenden Übergang an erfindungsgemäßen Ausführungsformen, d.h. es kann z.B. zügig in 15 min unter teilweiser Wärmeabführung bis maximal 40°C zugegeben werden, oder es kann z.B. über 2 h dosiert werden, dabei aber nur geringfügig gekühlt werden, wodurch man zunächst einen Temperaturanstieg auf 30°C zulässt und bei dieser Temperatur hält.
Besonders bevorzugt ist die Dosierung bei einer konstanten Temperatur.

Vorzugsweise werden im ersten Schritt auf 100 Gewichtsteile Wasser 5 bis 43 Gewichtsteile, vorzugsweise 11 bis 34 Gewichtsteile, insbesondere 13 bis 25 Gewichtsteile Trialkoxysilan eingesetzt.

Die Durchmischung im ersten Schritt kann durch einen statischen Mischer oder bevorzugt durch einen Rührer erfolgen.

Vorzugsweise wird nach Dosierung des Trialkoxysilans 5 min bis 5 h, besonders bevorzugt 10 min bis 3 h insbesondere 15 min bis 1,5 h nachgerührt. Die Nachrührzeit wird vorzugsweise so gewählt, dass die Summe der Zugabezeit des Silans und der Nachrührzeit 6 h nicht überschreiten.

Die Temperatur beim Nachrühren beträgt bei 0°C bis 60°C, bevorzugt bei 10°C bis 50°C, besonders bevorzugt bei 10°C bis 40°C, ganz besonders bevorzugt bei 10 bis 30 °C, insbesondere bei 15 bis 25 °C gehalten. Vorzugsweise ist die Differenz der Temperatur der Umsetzung im ersten Schritt und der Temperatur beim Nachrühren kleiner 20 °C, bevorzugt kleiner 10 °C, insbesondere kleiner 5 °C.

Vorzugsweise wird im zweiten Schritt die Base ausgewählt aus Alkalihydroxid, Erdalkalihydroxid, Alkalimethanolat, Ammoniak und organischen Aminen. Bevorzugte organische Amine sind Alkylamine, wie Mono-, Di- oder Triethylamin, Mono-, Di- oder Trimethylamin oder 1,2-Ethylendiamin. Bevorzugt werden die Hydroxide von Li, Na, K eingesetzt. Bevorzugt wird eine Lösung von Alkalihydroxid in Wasser oder in einem Alkanol mit 1 bis 3 Kohlenstoffatomen eingesetzt. Bevorzugte Alkanole sind 1-Propanol, 2-Propanol, Ethanol und insbesondere Methanol. Eine Lösung von Ammoniak oder Alkalihydroxid in Wasser ist ebenfalls bevorzugt. Geeignet sind verdünnte oder konzentrierte Lösungen von Alkalihydroxid 0,001 bis 1100 g/l bei 20 °C, bevorzugt 0,01 bis 500 g/l, besonders bevorzugt 0,1 bis 500 g/l.

Beim Einsatz einer Lösung von Alkalihydroxid in einem Alkanol mit 1 bis 3 Kohlenstoffatomen haften die Partikel weniger aneinander, zeigen einen niedrigeren Agglomerationsgrad und neigen weniger zum Verklumpen. Die Partikel zeigen ein in kosmetischen Anwendungen bevorzugtes trockneres Hautgefühl.

KOH ist als Alkalihydroxid bevorzugt.

Alternativ zu NaOH und KOH ist auch der Einsatz eines NaOH- oder KOH-Bildners möglich, der in zweiten Schritt mit dem im Hydrolysat vorhandenen Wasser sofort zu NaOH oder KOH reagiert.

Beispiele dafür sind Natriummethanolat, Kaliummethanolat, NaH und KH. Bei dieser Ausführungsform ist der Einsatz von Natriummethanolat oder Kaliummethanolat in methanolischer Lösung bevorzugt.

Vorzugsweise wird so viel Lösung von Base zugegeben, dass ein pH Wert von mindestens 6, vorzugsweise mindestens 6,5 und höchstens 10, vorzugsweise höchstens 9,5 erreicht wird, jeweils direkt nach Zugabe der Base gemessen. Durch die Zugabe der Menge an Base kann die Partikelgröße beeinflusst werden, wobei niedrige pH Werte größere Partikel ergeben. Der insbesondere bevorzugte pH Wert beträgt 7,5 bis 9.

Die Lösung von Base wird vorzugsweise innerhalb von 10 Sekunden bis 10 Minuten, insbesondere innerhalb von 1 bis 3 Minuten zugegeben, vorzugsweise unter starkem und kurzem Rühren.

Die Temperatur der Zugabe von Base im zweiten Schritt wird in einer bevorzugten Ausführungsform vorzugsweise bei 0°C bis 60°C, bevorzugt bei 10°C bis 50°C, besonders bevorzugt 10°C bis 40°C, ganz besonders bevorzugt bei 10°C bis 30°C, insbesondere bei 15°C bis 25°C gehalten. Vorzugsweise ist die Differenz der Temperatur beim Nachrühren und der Temperatur Zugabe von Base kleiner 20 °C, bevorzugt kleiner 10 °C, insbesondere kleiner 5 °C.

Die Durchmischung im zweiten Schritt kann durch einen statischen Mischer oder bevorzugt durch einen Rührer erfolgen.

Nach dem zweiten Schritt wird die Durchmischung vorzugsweise innerhalb von 10 Minuten, bevorzugt innerhalb von 5 Minuten beendet. Nach dem zweiten Schritt wird die Mischung vorzugsweise mindestens 1 h, bevorzugt mindestens 1,5 h, besonders bevorzugt mindestens 2,5 h nicht bewegt. Danach kann ein Rührer bei niedriger Drehzahl zugeschaltet werden, um ein Sedimentieren der Partikel zu verhindern. Dies ist optional und nicht notwendig, da sich die sedimentierten Polysilsesquioxanpartikel problemlos aufrühren lassen.

Nach dem zweiten Schritt wird die Temperatur der Mischung vorzugsweise mindestens 1 h, bevorzugt mindestens 1,5 h, besonders bevorzugt mindestens 2,5 h nicht mehr als 20 °C, bevorzugt nicht mehr als 10 °C verändert.

Wenn in der Anfangsphase im dritten Schritt, in welchem die Ausbildung der Partikel erfolgt, bewegt wird, treten vermehrt verformte, verwachsene oder agglomerierte Partikel auf.

In einer bevorzugten Ausführungsform wird die Mischung im dritten Schritt bis zur Isolierung der Polysilsesquioxanpartikel nicht bewegt.

Bevorzugt wird die Mischung im dritten Schritt mindestens 4 h, besonders bevorzugt mindestens 7 h, insbesondere mindestens 10 h aufbewahrt, bevor die Polysilsesquioxanpartikel isoliert werden. Auch Aufbewahrungszeiten bis 12 Wochen sind möglich.

Eine Trübung ist meist bereits nach 1 - 30 Minuten zu sehen.

Die Temperatur im dritten Schritt beträgt vorzugsweise 0°C bis 60°C, bevorzugt bei 10°C bis 50°C, besonders bevorzugt 10°C bis 40°C, ganz besonders bevorzugt 10°C bis 30°C, insbesondere 15°C bis 25°C. Bei niedrigen Temperaturen bilden sich größere Partikel, bei höheren Temperaturen bilden sich kleinere Partikel.

Bei einer Temperatur von 15°C bis 25°C besteht ein geringer oder kein Temperaturgradient der Reaktionsmischung zum Außenbereich, dadurch minimaler Wärmegradient zwischen Reaktorwandung und Reaktionslösung und dadurch minimierte thermische Konvektion während der Fällung der Partikel.

Das erfindungsgemäße Verfahren kann als Batchansatz, als Semi-Batch und oder als kontinuierlicher Prozess geführt werden.

Die Mischung wird in einer bevorzugten Ausführungsform nach dem dritten Schritt durch Zugabe einer Säure neutralisiert.

In einer bevorzugten Ausführungsform werden die Partikel nach dem dritten Schritt isoliert, vorzugsweise durch Abfiltrieren oder Zentrifugieren. Nach dem Isolieren werden die Partikel vorzugsweise mit einer Waschflüssigkeit gewaschen, die vorzugsweise ausgewählt wird aus VE-Wasser, Methanol, Ethanol und Gemischen davon. Die gewaschenen Partikel werden mit einer Flüssigkeit, die vorzugsweise ausgewählt wird aus VE-Wasser, Methanol, Ethanol und Gemischen davon redispergiert. Die erhaltene Dispersion weist vorzugsweise 5 bis 50 Gew.-%, besonders bevorzugt 10 bis 35 Gew.-%, insbesondere 10 bis 25 Gew.-% Partikel auf.

Bei Verwendung von flüchtigen Basen, wie es viele organische Amine sind, wie z.B. Di- oder Triethylamin oder Ammoniak, wird vorzugsweise nicht gewaschen.

Vorteilhaft ist die Verwendung von Ammoniak als Base, denn hier ist sowohl die Base selbst, als auch das entstehende Salz der Base unter den Bedingungen der Sprühtrocknung flüchtig.

Aus der in Schritt 2 erhaltenen Mischung oder aus der durch Isolieren, Waschen und Redispergieren erhaltenen Dispersion wird ein trockenes, rieselfähiges Pulver im Sprühtrockner hergestellt. Je nach Alkoholgehalt der Mischung wird als Trockengas Luft oder Inertgas, z.B. Stickstoff, Argon, Helium, Magerluft mit maximal 2% Sauerstoff eingesetzt. Die Sprühtrocknung kann in beliebigen zur Sprühtrocknung von Flüssigkeiten geeigneten und bereits vielfach bekannten Vorrichtungen, beispielsweise solchen mit mindestens einer Zweistoffdüse, einer Hartmetall- beziehungsweise Hohlkegeldüse oder einer Drallzerstäuberdüse oder mit einer rotierenden Zerstäuberscheibe in einem erwärmten Trockengasstrom durchgeführt werden. Vorzugsweise beträgt die Eingangstemperatur des Trockengasstroms, wobei es sich bevorzugt um Luft, Magerluft oder Stickstoff handelt, in die Sprühtrocknungs-Vorrichtung 110°C bis 350°C, besonders bevorzugt mindestens 150°C und höchstens 300°C, insbesondere mindestens 200°C und höchstens 280°C. Die Austrittstemperatur des beim Trocknen gebildeten Gasstroms beträgt vorzugsweise 40 bis 200°C, insbesondere 100 bis 180°C. Der Sprühdruck liegt vorzugsweise bei mindestens 500 hPa, besonders bevorzugt bei mindestens 800 hPa, höchstens bei 500 000 hPa, insbesondere höchstens bei 10 000 hPa. Die Umdrehung der Zerstäuberdüse liegt vornehmlich zwischen 4000 und 50 000 U/min.

In einer besonderen Ausführungsform werden die sprühgetrockneten Polysilsesquioxanpartikel nachgetrocknet, beispielsweise in einem Schaufeltrockner, Wirbelschichttrockner, Hordentrockner, Stromtrockner oder Trommeltrockner.

Partikel welche lange Zeit bei 110 °C Austrittstemperatur getrocknet werden, sind zwar Trocken, weisen aber einen hohen Si-OH Gehalt auf. Bei 140 °C ist der Si-OH Gehalt deutlich reduziert, aber noch nicht vollständig entfernt, bei 160 °C werden Si-OH Gruppen nochmals signifikant reduziert. Durch einen reduzierten Si-OH Gehalt ergeben sich Vorteile im Verteilungsverhalten und der Fluidisierung der Partikel.

Die Polysilsesquioxanpartikel zeigen bei der Untersuchung im Elektronenmikroskop vorzugsweise eine kugelförmige Gestalt. Die sphärischen Polysilsesquioxanpartikel weisen vorzugsweise eine durchschnittliche Sphärizität y von mindestens 0,6, insbesondere mindestens 0,7 auf. Die sphärischen Polysilsesquioxanpartikel weisen vorzugsweise eine durchschnittliche Rundheit x von mindestens 0,6, insbesondere mindestens 0,7 auf. Die Rundheit x und Sphärizität y können nach DIN EN ISO 13503-2, Seite 37, Annex B.3, insbesondere Figur B.1 bestimmt werden.

Vorzugsweise werden alle Verfahrensschritte beim Druck der umgebenden Atmosphäre, also etwa 0,1 MPa (abs.) ausgeführt; sie können auch bei höheren oder niedrigeren Drücken durchgeführt werden. Bevorzugt sind Drücke von mindestens 0,08 MPa (abs.) und besonders bevorzugt mindestens 0,09 MPa (abs.), besonders bevorzugt höchstens 0,2 MPa (abs.), insbesondere höchstens 0,15 MPa (abs.).

Alle vorstehenden Symbole der vorstehenden Formeln weisen ihre Bedeutungen jeweils unabhängig voneinander auf. In allen Formeln ist das Siliciumatom vierwertig.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

**Beispiele**

Volumengewichtete Partikelgrößenverteilung $d_{50}$

Die Bestimmung der volumengewichteten Partikelgrößenverteilung erfolgt nach ISO 13320 mittels statischer Laserstreuung mit dem Gerät Sympatec HELOS mit Trockendispergierer RODOS mit 2 bar Druckluft als Dispergiermedium. Der $d_{50}$ gibt dabei die mittlere Partikelgröße an.

Siebanalyse:

Die Siebanalyse erfolgte mittels Trockensiebung an einer Analysensiebmaschine vom Typ Retsch AS 200 basic bei 100 % Amplitude. Für die Analyse wurden vier Siebe nach DIN ISO 3310 folgender Maschenweite gestapelt: 200 $\mu$m, 100 $\mu$m, 40 $\mu$m, 20 $\mu$m, Boden. Es wurden jeweils 50 g Substanz auf dem obersten Sieb (200 $\mu$m) aufgebracht und für 10 Minuten gesiebt.

Bestimmung der Fluidisierung und Volumenzunahme:

In einen 50 mL PP-Zentrifugenröhrchen werden 6,0 g Polysilsesquioxanpartikel eingebracht, 30 Sekunden kräftig aufgeschüttelt und 1 h auf einer ebenen Fläche stehen gelassen. Danach wird, wenn nötig, durch leichtes Klopfen eine ebene Oberfläche erzeugt. Das Volumen(abgesetzt) der Probe wird abgelesen. Das Gebinde wird verschlossen und mindestens 30 Sekunden kräftig geschüttelt, bis sämtliches Material dispergiert ist. Das Zentrifugenröhrchen wird unmittelbar auf die Fläche zurückgestellt und sofort danach das Volumen(aufgeschüttelt) abgelesen. Aufschütteln und Ablesen wird insgesamt drei Mal wiederholt und aus den ermittelten Werten der Durchschnittswert Volumen (aufgeschüttelt, Durchschnittswert aus drei Versuchen) ermittelt. Die Volumenzunahme dann wird mit folgender Formel berechnet:

```
Volumenzunahme = Volumen (aufgeschüttelt, Durchschnittswert aus

drei Versuchen) x 100 / Volumen(abgesetzt)
```

Die mikroskopischen Untersuchungen wurden mit einem Rasterelektronemikroskop Zeiss SUPRA 55 VP durchgeführt. Die Proben wurden vor der Untersuchung mit einem Sputtercoater CCU-010 der Firma Safematic zur Verhinderung von Aufladungsphänomenen mit Gold besputtert.
Die sphärischen Polysilsesquioxanpartikel der Beispiele 1 bis 3 weisen eine durchschnittliche Sphärizität y von 0,8 und eine durchschnittliche Rundheit x von 0,85 auf nach DIN EN ISO 13503-2, Seite 37, Annex B.3, Figur B.1

**Beispiele**

Beispiel 1

1328 g vollentsalztes Wasser mit Leitfähigkeit 0,1 $\mu$S/cm wird in einem Glaskolben vorgelegt und auf 20 °C temperiert. Dabei wird mit 300 Upm gerührt. Der pH wird durch Zugabe von 0,1 molarer Salzsäure auf einen Wert von 4,40 eingestellt. 291,6 g Methyltrimethoxysilan werden über 1 h zudosiert, dabei wird die Temperatur auf 20 °C gehalten. Nach Ende der Dosierung wird 1 h bei 20 °C gerührt. Es werden 65,49 g 0,1 molare methanolische KOH-Lösung innerhalb 1 min bei 20 °C zugeben und insgesamt 3 min homogen vermischt. Dann wird der Rührer abgestellt. Nach 21 h werden die ausgefallenen Partikel abfiltriert, mit VE-Wasser gewaschen und mit VE-Wasser zu einer 15%-igen Suspension redispergiert. Anschließend wird mit einem Niro Mobile Minor Labor-Sprühtrockner bei 260 °C Eingangstemperatur und 155-160 °C Austrittstemperatur und Stickstoff als Trockengas getrocknet.

Beispiel 2

Die Durchführung erfolgt nach Verfahren von Beispiel 1, jedoch werden zur Fällung 85,14 g 0,1 molare methanolische KOH -Lösung zugegeben.

Beispiel 3

Die Durchführung erfolgt nach Verfahren von Beispiel 1, jedoch werden zur Fällung 7,96 g 1 molare wäßrige Ammoniaklösung zugegeben.

Vergleichsbeispiel V1

Die Durchführung erfolgt nach Verfahren von Beispiel 1, jedoch werden die ausgefallenen Partikel abfiltriert, mit VE-Wasser gewaschen und bei 150 °C 18 h im Trockenschrank getrocknet.

Vergleichsbeispiel 2

Die Partikel aus dem nicht-erfindungsgemäßen Vergleichsbeispiel V1 werden in einer Fließbett-Gegenstrahlmühle vom Typ Alpine 100AFG bei 5 bar Druck und einer Sichterdrehzahl von 4000 U/min dispergiert.

Vergleichsbeispiel V3

Die Durchführung erfolgt nach Verfahren von Beispiel 3, jedoch werden die ausgefallenen Partikel abfiltriert, mit VE-Wasser gewaschen und bei 150 °C 18 h im Trockenschrank getrocknet.

Vergleichsbeispiel 4

Die Partikel aus dem nicht-erfindungsgemäßen Vergleichsbeispiel V3 werden in einer Fließbett-Gegenstrahlmühle vom Typ Alpine 100AFG bei 7 bar Druck und einer Sichterdrehzahl von 12000 U/min dispergiert.

Tabelle 1

| Beispiel | | 1 | 2 | 3 | V1* | V2* | V3* | V4* |
|---|---|---|---|---|---|---|---|---|
| Fällungsbase | | KOH (MeOH) | KOH (MeOH) | Ammoniak | KOH (MeOH) | KOH (MeOH) | Ammoniak | Ammoniak |
| Fällungszeit** | Std. | 21 | 21 | 21 | 21 | 21 | 21 | 21 |
| Mahlung | | nein | nein | nein | nein | ja | nein | ja |
| Sprühtrocknung | | ja | ja | ja | nein | nein | nein | nein |
| Siebfraktion | <20 $\mu$m | 81 | 80 | 35 | 67 | 78 | 1 | 35 |
| Siebfraktion | 20 - 40 $\mu$m | 7 | 6 | 54 | 17 | 15 | 16 | 55 |
| Siebfraktion | 40 - 100 $\mu$m | 9 | 9 | 6 | 9 | 4 | 41 | 5 |
| Siebfraktion | 100 - 200 $\mu$m | 2 | 3 | 3 | 5 | 2 | 27 | 3 |
| Siebfraktion | >200 $\mu$m | 1 | 2 | 2 | 3 | 1 | 15 | 2 |
| Summe Anteil <40 $\mu$m | | 88 | 86 | 89 | 84 | 93 | 17 | 90 |
| Summe Anteil >100 $\mu$m | | 3 | 5 | 5 | 8 | 3 | 68 | 5 |
| Mittlere Partikelgröße $d_{50}$ | $\mu$m | 6,12 | 4,14 | 4,22 | 5,51 | 5,51 | 4,22 | 4,22 |

\* nicht erfindungsgemäß
\** Haltezeit in Stunden nach Basenzugabe

EP 3 377 559 B1

**Patentansprüche**

1. Verfahren zur Herstellung sphärischer Polysilsesquioxanpartikel, bei dem in einem ersten Schritt Trialkoxysilan der allgemeinen Formel (I)

$$RSi(OR^1)_3 \qquad (I),$$

in der

R einen Kohlenwasserstoffrest mit 1 bis 16 Kohlenstoffatomen dessen Kohlenstoffkette durch nicht benachbarte Gruppen -O-unterbrochen sein kann,
$R^1$ einen $C_1$- bis $C_4$-Alkylrest bedeuten, mit angesäuertem Wasser mit einem pH-Wert von höchstens 6 unter Durchmischung zu einem Hydrolysat umgesetzt wird, in einem zweiten Schritt das Hydrolysat mit einer Lösung einer Base in Wasser oder $C_1$- bis $C_4$-Alkanol vermischt wird,

in einem dritten Schritt die Mischung mindestens 2 h aufbewahrt wird
und in einem vierten Schritt die Polysilsesquioxanpartikel sprühgetrocknet werden.

2. Verfahren nach Anspruch 1, bei dem R Ethylrest oder Methylrest bedeutet.

3. Verfahren nach einem oder mehreren der voranstehenden Ansprüche, bei dem $R^1$ einen Ethylrest oder Methylrest bedeutet.

4. Verfahren nach einem oder mehreren der voranstehenden Ansprüche, bei dem im ersten Schritt auf 100 Gewichtsteile Wasser 5 bis 43 Gewichtsteile Trialkoxysilan eingesetzt werden.

5. Verfahren nach einem oder mehreren der voranstehenden Ansprüche, bei dem im zweiten Schritt eine Lösung von Ammoniak in Wasser oder Alkalihydroxid in einem Alkanol mit 1 bis 3 Kohlenstoffatomen eingesetzt wird.

6. Verfahren nach einem oder mehreren der voranstehenden Ansprüche, bei dem so viel Lösung von Base zugegeben wird, dass ein pH Wert von 6,5 bis 9,5 erreicht wird, jeweils direkt nach Zugabe von Base gemessen.

7. Verfahren nach einem oder mehreren der voranstehenden Ansprüche, bei dem die Temperatur der Zugabe von Base im zweiten Schritt 10°C bis 40°C beträgt.

8. Verfahren nach einem oder mehreren der voranstehenden Ansprüche, bei dem die Mischung im dritten Schritt mindestens 7 h aufbewahrt wird.

9. Verfahren nach einem oder mehreren der voranstehenden Ansprüche, bei dem die Mischung nach dem dritten Schritt durch Zugabe einer Säure neutralisiert wird.

10. Verfahren nach einem oder mehreren der voranstehenden Ansprüche, bei dem die Partikel nach dem dritten Schritt isoliert werden, mit einer Waschflüssigkeit gewaschen werden und die gewaschenen Partikel mit einer Flüssigkeit redispergiert werden.

11. Verfahren nach einem oder mehreren der voranstehenden Ansprüche, bei dem die Eingangstemperatur des Trockengasstroms in die Sprühtrocknungs-Vorrichtung 110°C bis 350°C beträgt.

**Claims**

1. Process for producing spherical polysilsesquioxane particles in which in a first step triakloxysilane of general formula (I)

$$RSi(OR^1)_3 \qquad (I),$$

in which

R represents a hydrocarbon radical having 1 to 16 carbon atoms whose carbon chain may be interrupted by nonadjacent -0- groups,

$R^1$ represents a $C_1$- to $C_4$-alkyl radical, is reacted with acidified water having a pH of not more than 6 with commixing to afford a hydrolyzate, in a second step the hydrolyzate is mixed with a solution of a base in water or $C_1$- to $C_4$-alkanol,

in a third step the mixture is stored for at least 2 h

and in a fourth step the polysilsesquioxane particles are spray-dried.

2. Process according to Claim 1, in which **R** represents ethyl radical or methyl radical.

3. Process according to one or more of the preceding claims, in which $R^1$ represents an ethyl radical or methyl radical.

4. Process according to one or more of the preceding claims, in which in the first step 5 to 43 parts by weight of trialkoxysilane are added per 100 parts by weight of water.

5. Process according to one or more of the preceding claims, in which in the second step a solution of ammonia in water or alkali metal hydroxide in an alkanol having 1 to 3 carbon atoms is employed.

6. Process according to one or more of the preceding claims, in which sufficient solution of base is added to ensure that a pH of 6.5 to 9.5 is achieved, in each case measured immediately after addition of base.

7. Process according to one or more of the preceding claims, in which the temperature of the addition of base in the second step is 10°C to 40°C.

8. Process according to one or more of the preceding claims, in which in the third step the mixture is stored for at least 7 h.

9. Process according to one or more of the preceding claims, in which after the third step the mixture is neutralized by addition of an acid.

10. Process according to one or more of the preceding claims, in which after the third step the particles are isolated and washed with a washing liquid, and the washed particles are redispersed with a liquid.

11. Process according to one or more of the preceding claims, in which the entry temperature of the dry gas stream into the spray-drying apparatus is 110°C to 350°C.

**Revendications**

1. Procédé de fabrication de particules sphériques de polysilsesquioxane, selon lequel, lors d'une première étape, un trialcoxysilane de la formule générale (I) :

$$RSi(OR^1)_3 \qquad (I)$$

dans laquelle

R signifie un radical hydrocarboné de 1 à 16 atomes de carbone dont la chaîne carbonée peut être interrompue par des groupes -0- non voisins,

$R^1$ signifie un radical alkyle en $C_1$ à $C_4$,

est mis en réaction avec de l'eau acidifiée à un pH d'au plus 6 avec mélange pour former un hydrolysat, lors d'une deuxième étape, l'hydrolysat est mélangé avec une solution d'une base dans de l'eau ou un alcanol en $C_1$ à $C_4$,

lors d'une troisième étape, le mélange est conservé pendant au moins 2 h,

et lors d'une quatrième étape, les particules de polysilsesquioxane sont séchées par pulvérisation.

2. Procédé selon la revendication 1, selon lequel **R** signifie un radical éthyle ou un radical méthyle.

3. Procédé selon une ou plusieurs des revendications précédentes, selon lequel $R^1$ signifie un radical éthyle ou un radical méthyle.

4. Procédé selon une ou plusieurs des revendications précédentes, selon lequel, lors de la première étape, 5 à 43 parties en poids de trialcoxysilane sont utilisées pour 100 parties en poids d'eau.

5. Procédé selon une ou plusieurs des revendications précédentes, selon lequel, lors de la deuxième étape, une solution d'ammoniac dans de l'eau ou d'un hydroxyde alcalin dans un alcanol de 1 à 3 atomes de carbone est utilisée.

6. Procédé selon une ou plusieurs des revendications précédentes, selon lequel suffisamment de solution de base est ajoutée pour qu'une valeur de pH de 6,5 à 9,5 soit atteinte, à chaque fois mesurée directement après l'ajout de la base.

7. Procédé selon une ou plusieurs des revendications précédentes, selon lequel la température de l'ajout de la base lors de la deuxième étape est de 10 °C à 40 °C.

8. Procédé selon une ou plusieurs des revendications précédentes, selon lequel le mélange est conservé pendant au moins 7 h lors de la troisième étape.

9. Procédé selon une ou plusieurs des revendications précédentes, selon lequel le mélange est neutralisé par ajout d'un acide après la troisième étape.

10. Procédé selon une ou plusieurs des revendications précédentes, selon lequel les particules sont isolées après la troisième étape, lavées avec un liquide de lavage et les particules lavées sont redispersées avec un liquide.

11. Procédé selon une ou plusieurs des revendications précédentes, selon lequel la température d'entrée du courant de gaz de sec dans le dispositif de séchage par pulvérisation est de 110 °C à 350 °C.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 3970449 B
- JP H1045914 A
- JP 2003335860 A
- JP H06248081 A
- JP H0488023 A